# EUROPEAN PATENT SPECIFICATION

(11) **EP 1 185 178 B1**
(45) Date of publication and mention of the grant of the patent: **25.10.2006**
(21) Application number: 00925479.8
(22) Date of filing: 25.04.2000
(51) Int. Cl.: A01N 65/00, A01N 25/04, A01N 25/02, A61K 36/282, A61K 36/53, A61K 36/537, A61K 36/752

(54) **INSECTICIDAL COMPOSITIONS**
INSEKTIZIDE ZUSAMMENSETZUNGEN
COMPOSITIONS INSECTICIDES

(30) Priority: 23.04.1999 GB 9909469
(43) Date of publication of application: 13.03.2002
(73) Proprietor: Herbal Sciences International Ltd, Loughton Essex 1G10 3TQ (GB)
(72) Inventor: Wilkinson, John Alfred, Enfield, Middlesex EN3 4SF (GB)
(74) Representative: Baldock, Sharon Claire
(86) International application number: PCT/GB2000/001589
(87) International publication number: WO 2000/064265

(56) References cited:
- EP-A- 0 495 684
- WO-A-00/00213
- WO-A-94/09631
- WO-A-95/23815
- WO-A-96/14046
- WO-A-96/29332
- WO-A-96/37210
- WO-A-97/17944
- WO-A-97/47193
- WO-A-98/22152
- WO-A-99/37148
- CH-A- 688 787
- GB-A- 2 224 934
- GB-A- 2 341 091
- US-A- 4 379 168
- US-A- 4 933 371
- US-A- 5 519 017
- PATENT ABSTRACTS OF JAPAN vol. 1998, no. 10, 31 August 1998 (1998-08-31) & JP 10 120519 A (OSAKA SEIYAKU:KK), 12 May 1998 (1998-05-12)
- DATABASE WPI Week 8849, 1988 Derwent Publications Ltd., London, GB; AN 1988-349708 XP002042392 "Gel-type base material for fragrance composition-obtd by mixing nonionic cellulose deriv. with aq. gel." & JP 63 260956 A (LION CORP.), 27 October 1988 (1988-10-27)
- A. NUMATA ET AL.: "Antifeedants for the larvae of the Yellow Butterfly, Eurema hecabe, in Lycoris radiata" CHEM. PHARM. BULL., vol. 31, no. 6, 1983, pages 2146-2149, XP002148848
- DATABASE WPI Section Ch, Week 199226 Derwent Publications Ltd., London, GB; Class A96, AN 1992-211969 XP002148849 & JP 04 139104 A (ARAKAWA CHEM IND LTD), 13 May 1992 (1992-05-13) cited in the application
- DATABASE WPI Section Ch, Week 198005 Derwent Publications Ltd., London, GB; Class A97, AN 1980-08537C XP002148850 & JP 54 160739 A (DASUKIN FRANCHISE K), 19 December 1979 (1979-12-19)
- S. PERRUCCI & G. MACCHIONI: "Structure/activity relationship of some natural monoterpenes as acaricides against Psoroptes cuniculi." J. NATURAL PRODUCTS, vol. 58, no. 8, August 1995 (1995-08), pages 1261-1264, XP000916948
- C. REGNAULT-ROGER ET AL.: "Insecticidal effect of essential oils from mediterranean plants upon Acanthoscelides obtectus say (Coleoptera, Bruchidae), a pest of kidney bean." J. CHEM. ECOLOGY, vol. 19, no. 6, 1993, pages 1233-1244, XP000940842

## Description

The present invention relates to plant essential oils and extracts and their uses as insecticides both in medical and non-medical applications. In particular the invention relates to the oils and extracts of Spanish sage (*Salvia lavandulifolia;* syn. *S. lavandulaefolia*) and *S. officinalis* "petite feuille Banon" and other essential oils or plant extracts such as those from *Artemisia dracunculus* (Tarragon), *Citrus limon* (Lemon), *Juniperus communis* (Juniper), *Laurus nobilis* (Bay), *Myristica fragrans* (Nutmeg), *Criganum vulgare* (Oregano), *Piper cubeba* (Cubebs), *Aloysia gratissima* (Whitebrush) and species of *Salvia* other than *S. lavandulifolia* which have the ability to kill a range of ectoparasites in a range of formulations.

Sucking lice *(Anuplura)* are a common pest of humans and animals which have a very wide global distribution, and are often spread by physical contact. In humans, lice are of two main genera. Lice of the genus *Pediculus* include head lice *(Pediculus humanus capitis* syn. *P. capitis)* and clothing lice *(P. humanus humanus* syn. *P. corporis*), whilst the commonest lice of the genus *Pthirius* are the crab (or public) lice, *Pthirius pubis.*

Head lice are a very common problem in children, from whom the insects can then be spread to other family members. Infestations are by no means limited solely to children. In the strictest sense, lice are obligate parasites, and hence are unlikely to leave a host voluntarily, yet whether infestations can be spread by inanimate objects, such as combs or hairbrushes remains a controversial issue. Louse infestations are usually accompanied by itching, although it is also possible that the insects could act as vectors for bacteria responsible for skin complaints, such as impetigo and scalp pyoderma (see Burgess (1995) *Advances in Parasitology* 36:271-342). In the UK, infestation rates were recorded as high as 23.1 % in primary school-aged children, and higher still (up to 30.3 %) in secondary schools. Other surveys suggest that one third of primary/junior school children are infested at least once per year (see Gratz (1997) *Human Lice- their prevalence, control and resistance to insecticides,* WHO).

There is evidence that a recrudescence of clothing lice, which are generally rarer than head lice in "developed" countries, is occurring (see Gratz (1997) *Human Lice- their prevalence, control and resistance to insecticides,* WHO). These insects have been linked to the transmission of trench fever, relapsing fever and typhus (see Van Der Lann and Smit (1996) *Nederlands Tijdschrift voor Geneeskunde* 140: 1912-1915).

Conventional treatment of head lice depends on application of insecticidal compositions and/or physical removal of lice and nits (lice eggs) by mechanical means (usually a fine-toothed comb). Insecticides used for treatment of head lice include organophosphates (e.g. malathion), pyrethroids, lindane and DDT. There are a number of problems associated with these compounds, including health concerns over exposure to organophosphates, development of resistance by lice (see Gratz (1997) Human *Lice- their prevalence, control and resistance to insecticides,* WHO) and an undesirable product formulation (e.g. unpleasant smell). Some preparations also contain very high (>80% v/v) concentrations of alcohol, which has been linked to instances of allergenic reactions.

A number of plant-derived treatments for lice have been developed previously, ranging from Stemosa *tuberosa* and *Hyssop officinalis* extracts, quassia chips, pyrethrins, rotenone (from *Derris* or *Lonchocarpus* spp.) to a number of essential oils. Essential oils which have been used, or suggested for use against lice, or which have been studied scientifically include *Pimpinella anisum* (Aniseed), *Cinnamomum zeylanicum* (Cinnamon), *Cymbopogon nardus* (Citronella), *Eucalyptus* spp. (Eucalyptus), *Pelargonium graveolens* (Geranium), *Hyssop officinalis* (Hyssop), *Juniperus communis* (Juniper), *Lavandula officinalis* (Lavender), *Citrus limon* (Lemon), *Cypressus* x *leylandii* (Leyland cypress), *Myrtus communis* (Myrtle), *Myristica fragrans* (Nutmeg), *Origanum vulgare* (Oregano), *Mentha x piperita* (Peppermint), *Pinus sylvestris* (Pine), *Thymus zygis* (Red thyme), *Rosemarinus officinalis* (Rosemary), *Melaleuca alternifolia* (Tea tree) and *Cananga odorata* (Ylang ylang . A number of these preparations have, however, been found to be of limited efficacy or have been associated with mammalian toxicity (see Burgess (1995). *Advances in Parasitology* 36:271-342). US patents 5,227,163 and 5,411,992 to Eini claim that a number of essential oils, including sage, and also a range of terpenoid compounds possess lice-repellent activity. Rosemary and Eucalyptus essential oils have similarly been demonstrated to possess repellent activity towards clothing lice (see Mumcuoglu et al (1996) *Entomologia Experimentalis* et *Applicata* 78: 309-314). Patent Application GB 2,341,091 also describes eucalyptus essential oil, in combination with lavender and tea-tree essential oils, as a treatment for head lice. Combinations of essential oils, including rosemary, eucalyptus, tea-tree and lavender, as well as those from a number of other species, are also proposed as compositions suitable for lice treatment (WO00/00213).

Ectoparasites of livestock, such as sheep, can detrimentally affect productivity of milk and meat, and the quality of wool and leather. Additionally, the welfare of animals infested with parasites can be seriously affected (see Bates (1999) in: Martin and Aitken (editors), *Diseases of Sheep* 3rd Ed., Chapter 45, Blackwell Science). Conventional treatment and control regimes are expensive to implement, and can be associated with health risks to the farmer.

Sheep scab results from infestation by *Psoroptes ovis* (sheep scab mite), and is usually treated by plunge dipping in washes containing organophosphates (such as diazinon or propetamphos) or synthetic pyrethroids (flumethrin or cypermethrin). Organophosphate dips have the advantage that a single immersion is sufficient for treatment (see Bates (1999) in: Martin and Aitken (editors), *Diseases of Sheep* 3rd Ed., Chapter 46, Blackwell Science), but have been linked to health concerns for farmers (e.g. see Rees (1996). *Occupational and Environmental Medicine* 53: 258-263), whereas pyrethroid dips need to be used twice at 14 day intervals for effective treatment. *P. ovis* is usually restricted to sheep, although Psoroptic mange in cattle has been reported, and is a major problem in mainland Europe and the United States of America. The ear mite, *P. cuniculi,* is a close relative of *P. ovis,* and is primarily associated with rabbit populations, although it has also been isolated from sheep, goats and horses.

Chewing lice *(Bovicula ovis)* are another major ectoparasite of sheep, which can severely affect the quality of fleeces and hides. Treatment of lice is by similar means to the scab mite, by dipping in solutions containing organophosphates or synthetic pyrethroids. Pour-on treatments based on synthetic pyrethroids have been developed, but in Australia, resistance developed to these products within a few years of their release on to the market (see Bates (1999) in: Martin and Aitken (editors), *Diseases of Sheep* 3rd Ed., Chapter 45, Blackwell Science). The lipophilic nature of the components of the compositions described in the present invention make them ideal for development as pour-on treatments, for which lipophilicity is a key feature.

Sage contains a rich variety of chemicals, many of them terpenes and terpenoids. They include amorphene, aromadendrene, borneol, bornyl acetate, cadinene, camphene, camphor, β-caryophyllene, caryophyllene oxide, 1,8-cineole (eucalyptol), copaene, cubebene, p-cymene, geraniol, germacrene D, gurjunene, α-humulene, limonene, linalool, linalyl acetate, manool, myrcene, ocimene, palustrol, phellandrene, α-pinene, β-pinene, sabinene, sabinyl acetate, spathulenol, terpenyl acetate, terpinen-4-ol, terpinene, terpineol, terpinolene, α-thujone, β-thujone, viridifloral. Additional components of sage extracts could include (1R,5R)-epoxysalvial-4(14)-en-1-one, (2R,5E)-epoxycaryophyll-5-en-12-al, (2R,5E)-epoxycaryophyll-5-ene, (2S,5E)-epoxycaryophyll-5-en-12-al, 3-octanol, acetic-acid-ester, α-bisabolol, α-terpineol, α-terpinyl-acetate, arachidic acid, benzaldehyde, β-gurjuene, β-myrcene, β-sitosterol, butyric acid, caprylic acid, cerotinic acid, cis-3-hexen-1-ol, cis-allo-ocimene, cis-beta-ocimene, cis-linalol-oxide, citral, citronellol, cuminaldehyde, delta-3-carene, furfurol, gamma-terpinene, geranial, geraniol, geranyl acetate, isospathulenol, lignoceric acid, linoleic acid, linolenic acid, n-nonanol, n-pentanol, neral, nerol, nerolidol, neryl acetate, oleanolic-acid, oleic-acid, palmitic acid, phellandrene, propionic acid-ester, rosmarinic acid, salvia-4(14)-en-1-one, sclareol, stearic acid, terpinen-4-ol, trans-allo-ocimene, trans-β-ocimene, trans-β-terpineol, trans-linalool oxide, ursolic acid, valeric acid-ester, 5-hydroxy-6,7,4'-trimethoxyflavone, 6,8-di-C-glucosylapigenin, 6-hydroxylutein-6,3'-dimethylether, 6-methoxy apigenin-7-glucoside, 6-methoxy apigenin-7-glucuronide, 6-methoxy luteolin-7-glucoside, 6-methoxy luteolin-7-glucuronide, apigenin-7-glucoside,apigenin-7-glucuronide, betulinic-acid, carnesol, chrysoeriol-7-glucuronide, luteolin-7-glucoside, luteolin-7-glucuronide, luteolin-7-glucuronide-3'-glucoside, luteolin diglucoside, picrosalvin, salvigenin, (E)-nerolidol, 2,6-dimethyl-10-(*p*-tolyl)-undeca-2,6-diene, 2-octanol, 3-octanol, allo-aromadendrene, α-amorphene, α-copaene, α-cubebene, α-gurjunene, α-humulene, α-muurolene, α-phellandrene, α-selinene, β-cyclocitral, cadina-1,4-diene, cis-α-bisabolene, δ-cadinene, γ-muurolene, isoamyl-acetate, isocaryophyllene, isopinocamphone, methyl perillate, myrtenol, myrtenyl acetate, perillaldehyde, perillyl acetate, perillyl alcohol, perillyl-butyrate, t-cadinol, trans-α-bergamotene, trans-calamenene and viridiflorene.

Sage essential oil samples which are most effective in killing parasites usually contain high concentrations of sabinene, p-cymene and/or terpinen-4-ol. Sabinene [(1R/S, 5R/S)-1-isopropyl-4-methylenebicyclo[3.1.0]hexane] is a bicyclic monoterpenoid, of the formula C₁₀H₁₆, p-cymene [1-propyl-4-methylbenzene] is a monocyclic monoterpene hydrocarbon, of the formula C₁₀H₁₄, and terpinen-4-ol [(S)-1-isopropyl-4-methyl-3-cyclohexen-1-ol] is a cyclic monoterpene alcohol of the formula C₁₀H₁₈O, whose respective formulae can be represented thus:

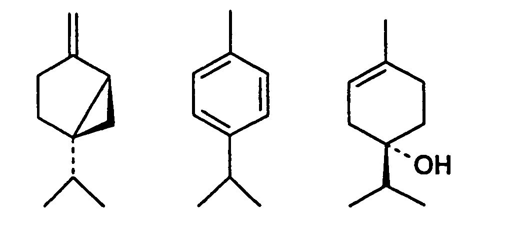

Other compounds tested in the present research include linalool [3,7-dimethyl-1,6-octadien-3-ol], limonene [(R/S)-4-isopropenyl-1-methylcyclohexane], 1,8-cineole [eucalyptol; 1,3,3-trimethyl-2-oxabicyclo[2.2.2]octane], camphor [1,7,7-trimethylbicyclo[2.2.1]heptan-2-one], a-terpineol [(R/S)-*p*-menth-1-en-8-ol], a-pinene [(1R/S, 5 R/S)-2,6,6-trimethyl-bicyclo[3.1.1]hept-2-ene], b-pinene [(1R/S, 5 R/S)-6,6-dimethyl-2-methylenebicyclo[3.1.1]heptane], borneol [endo-(1R/S)-1,7,7-trimethylbicyclo[2.2.1]heptan-2-ol], myrcene [7-methyl-3-methylen-1,6-octadiene], camphene [(1R/S)-2,2-dimethyl-3-methylenebicyclo[2.2.1]heptane] and b-caryophyllene [trans-(1R,9S)-8-methylene-4,11,11-trimethylbicyclo[7.2.0]undec-4-ene]. Their formulae can be represented thus:

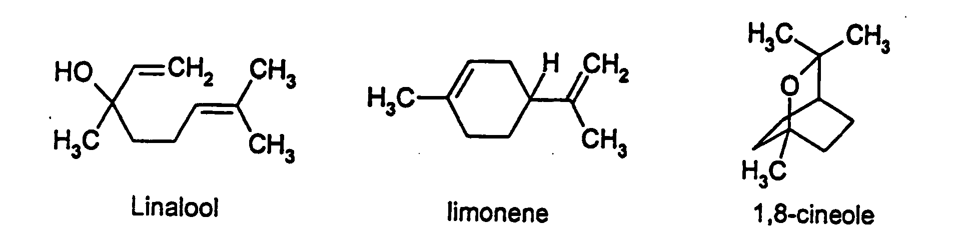

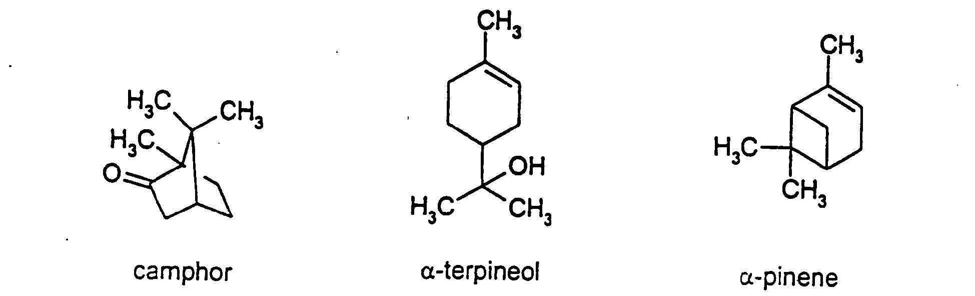

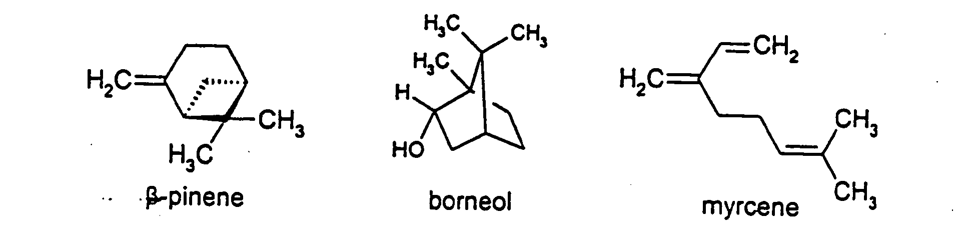

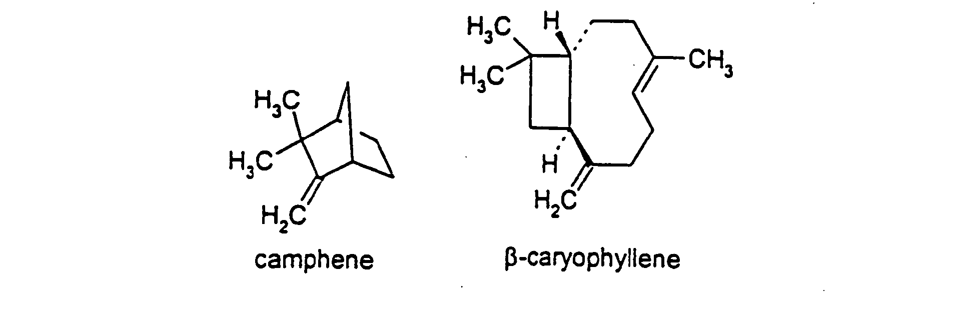

The parasite-killing activity of these compounds described in the present invention is previously unreported, although they have has been shown to possess other biological activities. For example, US patent 5,635,184 to Camano describes potent antibacterial activity of the essential oil of *Schinus molle,* which contains significant amounts of sabinene. A composition based on volatile monoterpene compounds, one of which could include sabinene, has been described as possessing activity against house- and leopard mites (see Arakawa Chem Ind Ltd (1992) Japanese Patent 04139104A).

It is also desirable for these oils to have low concentrations of thujone and sabinyl acetate, which have been linked to mammalian toxicity (see Tisserand (1995) *Essential Oil Sa fety- A Guide for Health Care Professionals;* Fournier *et al.* (1993) *Plant Medica* 59: 96-97). Spanish sage *(Salvia lavandulifolia)* is particularly desirable in this regard, due to its low thujone content, and is classified as safe to use, being unlikely to cause toxicity, irritation or sensitisation. Despite the presence of thujone in other Sage essential oils, these have been determined to be of lower toxicity than expected on the basis of their thujone content alone, and are unlikely to cause irritation or sensitisation (see Tisserand (1990) *Essential Oil Safety Data Manual).* The same source also indicates that, for dermal application, essential oils of similar potential toxicity as thujone-rich sage oil could be applied twice weekly at concentrations between 1-2 and 3-5 % by volume. These values are all above the final concentration of sage oil in an aqueous-alcoholic carrier, as described in the present invention.

CH 688787 discloses compositions comprising essential oils and uses thereof e.g. antiparasitic shampoos. JP 10120519 teaches an exterminator for parasitic insect pests comprising essential oils from different plants.

US 4,933,371 teaches a method for controlling ticks and fleas using a composition comprising linalool.

WO 96/37210 teaches pharmaceutical compositions based on etheric oils obtained from plants such as *Thyme* and *Saturea* and uses thereof. US 5, 519, 017 teaches the use of galanthamine for the treatment of alcoholism.

It is therefore an object of the present invention to provide treatments for parasitic infestations of humans and animals, which are pleasant to use, effective, of low mammalian toxicity, and are unlikely to lead to the development of side-effects or adverse reactions. It is a further object of the invention to provide compositions for treating insect parasitic infestations of fabrics including clothing and furnishings. It is yet a further object of the invention to provide compositions for treatment of parasitic infestations of plants.

In accordance with a first aspect the invention provides a composition comprising an essential oil in a gel carrier, said essential oil being obtained from a plant selected from the genera *Salvia, Artemisia, Citrus, Laurus, Myristica, Origanum, Piper* or *Aloysia,* said composition being for use in the treatment of a human or animal having a parasitic insect infestation.

The gel may be based on agar, agarose, gelatin, or a synthetic gelling agent and may be subject to dilution with water and/or alcohol, preferably, isopropyl alcohol. As used herein the term "synthetic gelling agent" refers to a material having the properties of a gel but which is not found in nature.

The above described compositions are useful for the killing and/or repelling of parasitic insects in a variety of different contexts. They may be used for treatment of humans having a parasitic insect infestation, for example head lice or pubic lice. The gel formulations of the invention are particularly useful for this purpose and may be formulated for topical use such as for hair gels or mousse, for example.

Further preferred embodiments of the compositions and methods of the invention are disclosed herein below and in the accompanying examples and claims.

The present invention is based on the discovery that essential oils can effectively kill insects, especially parasitic insects such as ectoparasites. Certain oils, including those from *Salvia* species and other plant species containing relatively high concentrations of sabinene p-cymene, β-pinene, myrcene or terpiner-4-ol have been found, surprisingly, to be most effective. and therefore of considerable commercial potential. Species of *Salvia* suitable for use in the present invention include *Salvia aethiopis, Salvia amissa, Salvia apiana, Salvia argentea, Salvia arizonica, Salvia azurea, Salvia ballotiflora, Salvia blodgettii, Salvia brandegei, Salvia carduacea, Salvia carnosa, Salvia chapmanii, Salvia chia, Salvia clevelandii, Salvia coccinea, Salvia columbariae, Salvia davidsonii, Salvia divinorum, Salvia dolichantha, Salvia dorrii, Salvia earlei, Salvia engelmannii, Salvia eremostachya, Salvia farinacea, Salvia funerea, Salvia glutinosa, Salvia grahamii, Salvia greatae, Salvia greggii, Salvia henryi, Salvia hispanica, Salvia lancifolia, Salvia lemmonii, Salvia leptophylla, Salvia leucophylla, Salvia longistyla, Salvia lycioides, Salvia lyrata, Salvia mellifera, Salvia micrantha, Salvia microphylla, Salvia misella, Salvia mohavensis, Salvia munzii, Salvia nemorosa, Salvia nutans, Salvia occidentalis, Salvia officinalis, Salvia pachyphylla, Salvia parryi, Salvia penstemonoides, Salvia pinguifolia, Salvia pitcheri, Salvia potus, Salvia pratensis, Salvia privoides, Salvia ramosissima, Salvia reflexa, Salvia regla, Salvia riparia, Salvia roemeriana, Salvia sclarea, Salvia serotina, Salvia sonomensis, Salvia spathacea, Salvia splendens, Salvia subincisa, Salvia summa, Salvia texana, Salvia thomasiana, Salvia tiliifolia, Salvia urticifolia, Salvia vaseyi, Salvia verbenacea, Salvia verticillata, Salvia vinacea, Salvia virgata, Salvia X bernardina, Salvia X palmeri, Salvia X superba and Salvia X sylvestris. Particularly preferred are Salvia lavandulifolia or S. officinalis.* Other species suitable for use in the present invention include *Artemesia dracumculus* (Tarragon), Citrus, in particular *Citrus limon* (lemon), *Juniperus communis* (Juniper), *Laurus nobilis* (Bay), *Myristica fragrans* (Nutmeg), *Origanum Vulga re* (Oregano) , *Piper cubeba* (Cubebs) and *Aloysia gratissima* (Whitebrush). The oils can be formulated into both aqueous and nonaqueous formulations which remain active against lice. The preferred formulation, in which the oil maintains significant activity at relatively low concentrations is based on a gel, in which the essential oil is dispersed. This has the advantage over mixed aqueous alcoholic formulations to which sensitive individuals may react adversely due to their alcohol content. In a mixed alcoholic-aqueous carrier, concentrations of oil required to kill human lice are comparable to those of the (organophosphate-based) active components in commercial head lice treatments.

The present invention therefore may be summarised as an effective dose of an essential oil or plant extract, preferably Spanish sage essential oil in the concentration range 0.1-50 % (w/v), in a gel formulation, such as Lubrajel TW (available from United Guardian Inc of 230, Marcus Blvd, Hauppauge, New York, 11788), containing water (0-66 % v/v), IPA (0-20 %) and/or additional plant extracts, as required. For example, the preferred formulation for treatment of human lice is Lubrajel TW diluted 1:2 (v/v) with water, to which 4 % (w/v) Spanish sage essential oil and 0.5 % (w/v) decolourised *Aloe vera* gel are added.

The ability of sabinene-containing essential oils, and in particular those from plants of the genus *Salvia* (sage), to kill insects, and in particular parasitic lice and mites is demonstrated in the examples herein. Essential oils, and in particular the oils from *lavandulifolia* and *S. officinalis* "petite feuille Banon", typically containing high concentrations of sabinene, and low concentrations of thujone, immobilise both head- and clothing lice almost immediately on contact, and remain highly active when appropriately formulated. As described above the present invention incorporates a number of embodiments comprising a range of gel forms suitable for topical application to the head or skin of humans, application to furnishings and clothing and to plants. The same preparations are also effective treatments for animal ectoparasites, for example sheep parasites, including *Psoroptes* (mite) and *Bovicula* (louse) species.

It is further demonstrated herein that a number of terpene and terpenoid components of the sage oil synergistically interact, and kill ectoparasites at lower doses than when applied individually in an in vitro model. (see Example 4).

The terpene hydrocarbon content of essential oil samples can be elevated by mixing with ethanol, and partitioning the resulting solution with known volumes of water. The most effective ratio of solvents for these experiments is 5 volumes of ethanol:water (3:2) per volume of essential oil, although a range of ratios, from 1:1:1 to 6:4:1 (ethanol:water:essential oil) are also effective. When the partitioning is repeated, the resulting oil shows an increased content of terpene hydrocarbons.

Previously, the lice-repellency activity of *Salvia sclarea* essential oil and terpene or terpenoid components characteristic of this, and related oils, has been described (see Eini et al. 1995 US Patent 5,411,992). The lice- and mite-killing activity of sage essential oils, and their chemical components is previously unreported.

Activity of essential oils and terpene or terpenoid components of essential oils was tested using an in vitro model system. Test solutions were prepared by accurately dissolving and mixing essential oils or terpenoid components in a range of carrier solutions, including water, isopropanol (propan-2-ol; IPA), water-IPA mixtures in the range 10-90 % by volume IPA and inactive vegetable oils, such as grape seed oil and a range of gel formulations, containing 0-66% water and/or 0-20% IPA. Clothing lice *(Pediculus humanus humanus),* head lice *(Pediculus humanus capitis)* or sheep biting lice *(Bovicula ovis)* were then transferred to small volumes (typically 1-2 ml) of these test solutions, in glass containers, and were left in contact with the solutions for 10 minutes. During this time the solutions were occasionally shaken gently to ensure adequate contact between the lice and the test solution. After 10 minutes, the lice were removed, and placed on filter paper to remove excess treatment.

Exposure of lice to test substances in this way (treatments or controls) resulted in the lice being temporarily immobilised. The lice were then monitored for signs of physical activity for a period of up to 90 minutes. Lice exposed to non-active test substances or controls typically regained activity within 5 minutes of their removal from the test solution; those which failed to regain any activity within a post-treatment period of 30-90 minutes were classified as dead.

Due to their smaller size, parasitic mites *(Psoroptes ovis* or *P. cuniculi*) were treated by adding droplets of test solution to the parasites. The time taken for the mites to stop moving in the test solution was taken as an indication of the toxicity of the test substance. In all other respects, the experiments were performed similarly to those used to study parasitic lice.

Each experiment was performed at a range of concentrations of the test substance, to determine the dose-response of these materials. Activity of the treatments was expressed as an LD₅₀, which was the concentration of essential oil or terpenoid compound sufficient to kill half of the parasites exposed to that treatment.

In the following non-limiting Examples reference is made to the following figures.
Figure 1 shows the effect of concentration of sage oil in vegetable oil carrier on clothing lice recovery (%);
Figure 2 shows the effect of concentration of sage oil in 20% (v/v) IPA-water on clothing lice recovery (%);
Figure 3 shows data obtained on testing activity on clothing lice of essential oils from a number of plant genera at 20 mg/ml (2% w/v) in an aqueous gel;
Figure 4 shows data obtained on testing activity on clothing lice of essential oils from a number of plant genera at 40 mgl (4% w/v) in an aqueous gel;

The invention will be more fully understood by reference to the following examples. Only the examples, with gels however, illustrate the embodiments of the invention. These examples are not to be construed to limit the scope of the invention.

### Example 1

Activity of sabinene-containing essential oils against human parasitic lice.

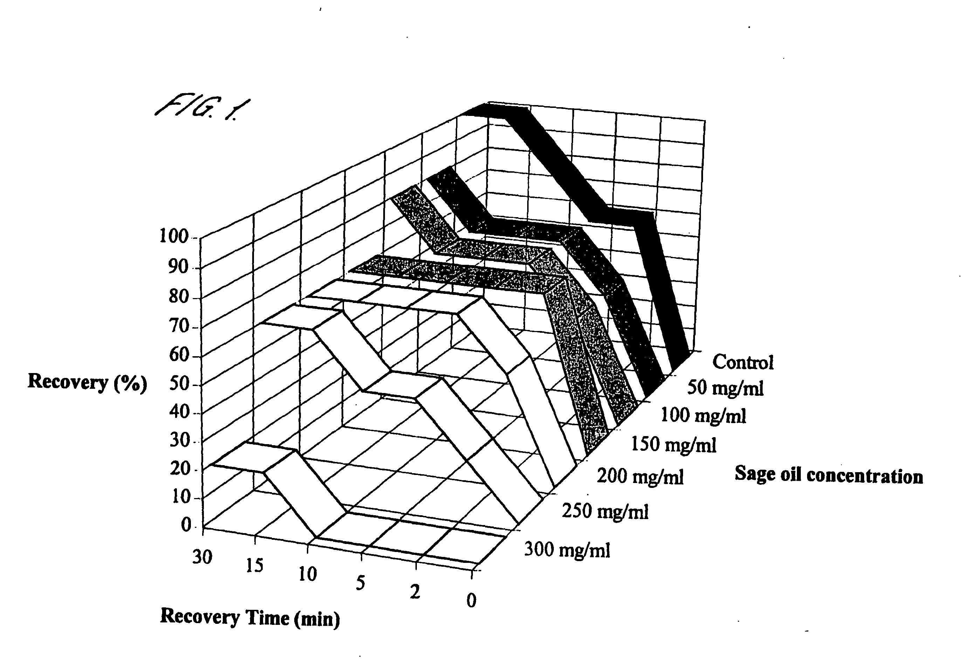

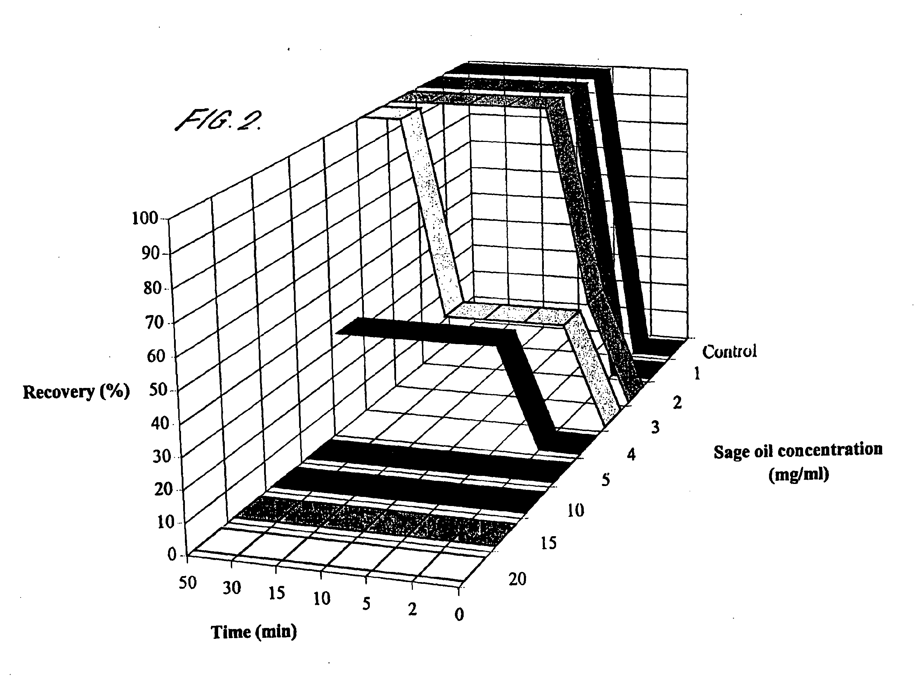

At high concentrations, sage essential oil led to gross morphological disruption of the human lice, characterised by abdominal swelling, and the development of an intense red coloration in the body and limbs. When dissolved in an inert (no activity against lice) carrier oil, the LD₅₀ for sage essential oil containing as little as 5% sabinene was in the concentration range of 250-300 mgml⁻¹ (Table 1, FIG. 1). In a carrier consisting of IPA (20% v/v) in water, however, the LD₅₀ of the same oil was much lower, at between 3-4 mgml⁻¹ (Table 2, FIG. 2). In gel-based formulations, LD₅₀ values were below 10 mgml^{- 1}, in the case of gel diluted 1:2 (v/v) with water (Table 3) or with undiluted gel supplemented with 20 % (w/v) IPA. The diluted gel was selected as the preferred formulation, due to the low LD₅₀ of sage essential oil it contains, and the absence of alcohol from the formulation, to which certain individuals can be sensitive. Specifically, the preferred formulation is based on Lubrajel TW (a synthetic gel based on glyceryl polymethacrylate and propylene glycol), diluted with deionized water 1:2 (v/v), to which the essential oil, and other components are added, as required.

As head lice *(Pediculus humanus capitis*) were slightly more sensitive to the sage oil formulations than clothing lice (*P. humanus humanus*) (Tables 2 and 4), subsequent experiments were performed using clothing lice as the model system, as concentrations of oils effective against these lice would also be effective against head lice.

**Table 1: Effect of concentration of sage oil in vegetable oil carrier on clothing lice recovery (%)**

| Concentration | Time post-treatment (minutes) | | | | | |
|---|---|---|---|---|---|---|
| (mgml⁻¹) | 0 | 2 | 5 | 10 | 15 | 30 |
| 0 | 0 | 60 | 80 | 80 | 100 | 100 |
| 50 | 0 | 40 | 60 | 60 | 60 | 80 |
| 100 | 0 | 40 | 60 | 60 | 60 | 80 |
| 150 | 0 | 60 | 60 | 60 | 60 | 60 |
| 200 | 0 | 40 | 60 | 60 | 60 | 60 |
| 250 | 0 | 20 | 40 | 40 | 60 | 60 |
| 300 | 0 | 0 | 0 | 0 | 20 | 20 |

**Table 2: Effect of concentration of sage oil in 20% (v/v) IPA-water on clothing lice recovery (%)**

| Concentration | Time post-treatment (minutes) | | | | | |
|---|---|---|---|---|---|---|
| (mg ml⁻¹) | 0 | 2 | 5 | 10 | 15 | 30 |
| 0 | 0 | 0 | 100 | 100 | 100 | 100 |
| 1 | 0 | 0 | 100 | 100 | 100 | 100 |
| 2 | 0 | 33 | 100 | 100 | 100 | 100 |
| 3 | 0 | 33 | 33 | 33 | 33 | 100 |
| 4 | 0 | 0 | 33 | 33 | 33 | 33 |
| 5 | 0 | 0 | 0 | 0 | 0 | 0 |
| 10 | 0 | 0 | 0 | 0 | 0 | 0 |
| 15 | 0 | 0 | 0 | 0 | 0 | 0 |
| 20 | 0 | 0 | 0 | 0 | 0 | 0 |

**Table 3: Effect of concentration of sage oil in gel diluted 1:2 with water on head lice recovery (%)**

| Concentration | Time post-treatment (min) | | | | |
|---|---|---|---|---|---|
| (mgml⁻¹) | 0 | 2 | 5 | 10 | 30 |
| 0 | 0 | 60 | 100 | 100 | 100 |
| 10 | 0 | 0 | 0 | 0 | 20 |
| 50 | 0 | 0 | 0 | 0 | 0 |
| 100 | 0 | 0 | 0 | 0 | 0 |

**Table 4: Effect of concentration of sage oil in 20% (v/v) IPA-water on head lice recovery (%)**

| Concentration | Time post-treatment (min) | | | | |
|---|---|---|---|---|---|
| (mgml⁻¹) | 0 | 2 | 5 | 10 | 30 |
| Control | 0 | 25 | 50 | 100 | 100 |
| 1 | 0 | 33 | 67 | 67 | 33 |
| 2 | 0 | 29 | 43 | 57 | 29 |
| 3 | 0 | 25 | 50 | 50 | 50 |
| 4 | 0 | 0 | 0 | 0 | 0 |
| 5 | 0 | 0 | 0 | 0 | 0 |
| 10 | 0 | 0 | 0 | 0 | 0 |

In the case of the gel-based formulations, addition of IPA had varying effects, depending on the gel strength. In strong gels (undiluted with water), activity increased with the addition of IPA over the range 0-20 % (v/v), but in weaker gels, (diluted 1:1 or 1:2 with water), addition of IPA over the same range reduced activity (Table 6).

**Table 6: Effect of gel strength and IPA content of formulations containing 10 mgml⁻¹ sage essential oil on clothing lice recovery after 30 minutes (%)**

| Gel strength | Lice recovery (%) | |
|---|---|---|
| | No IPA | 20 IPA (v/v) |
| Undiluted | 100 | 20 |
| Diluted 1:1 (v/v) | 60 | 60 |
| Diluted 1:2 (v/v) | 20 | 100 |

A number of essential oil samples were studied for activity, and data from gas chromatographic analysis of the most active oil samples (*S. officinalis* "petite feuille Banon" and Salvia lavandulifolia), is given in tables 7 and 8.

**Table 7: Identified chemical components of the essential oil of Salvia officinalis "petite feuille Banon"**

| Name | Percentage peak area |
|---|---|
| a-pinene | 4.26 |
| camphene | 5.61 |
| sabinene/β-pinene | 8.03 |
| myrcene | 3.16 |
| Iimonene/1.8-cineole/ *p*-cymene | 33.80 |
| linalool | 0.32 |
| a-thujone | 0.47 |
| b-thujone | 0.22 |
| camphor | 9.51 |
| borneol/isoborneol | 2.29 |
| terpinen-4-ol | 1.71 |
| a-terpineol | n.d. |
| linalyl acetate | n.d. |
| b-caryophyllene | 3.90 |

| | |
|---|---|
| n.d. Not determined | |

**Table 8: Identified chemical components of the essential oil of Salvia lavandulifolia**

| Name | Percentage peak area |
|---|---|
| a-pinene | 12.69 |
| camphene | 9.39 |
| sabinene/b-pinene | 2.34 |
| myrcene | 0.97 |
| limonene/1,8-cineole/ p-cymene | 37.46 |
| linalool | 0.78 |
| a-thujone | 0.17 |
| b-thujone | n.d. |
| camphor | 22.98 |
| borneol/isoborneol | 1.62 |
| terpinen-4-ol | 0.15 |
| a-terpineol | 1.08 |
| linalyl acetate | 1.35 |
| b-caryophyllene | 0.94 |

| | |
|---|---|
| n.d. Not determined | |

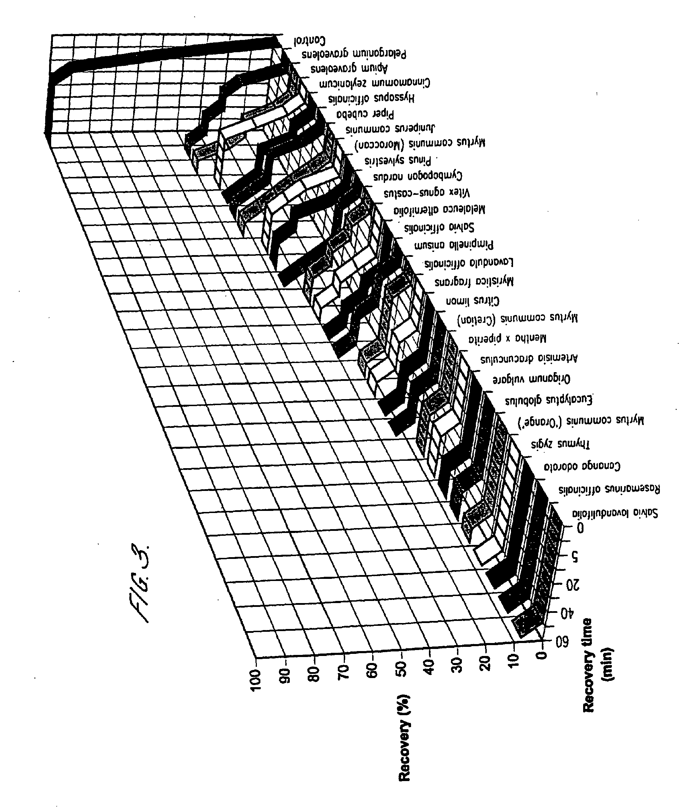

When incorporated into the preferred formulation, Spanish sage essential oil was more effective as a pediculicide than any of the other essential oils tested, including those known from the prior art. After exposure to sage essential oil at a concentration of 20 mgml⁻¹ (2 % w/v), clothing lice started a (very limited) recovery after 50 minutes, later than all the other oil samples tested (FIG. 3).

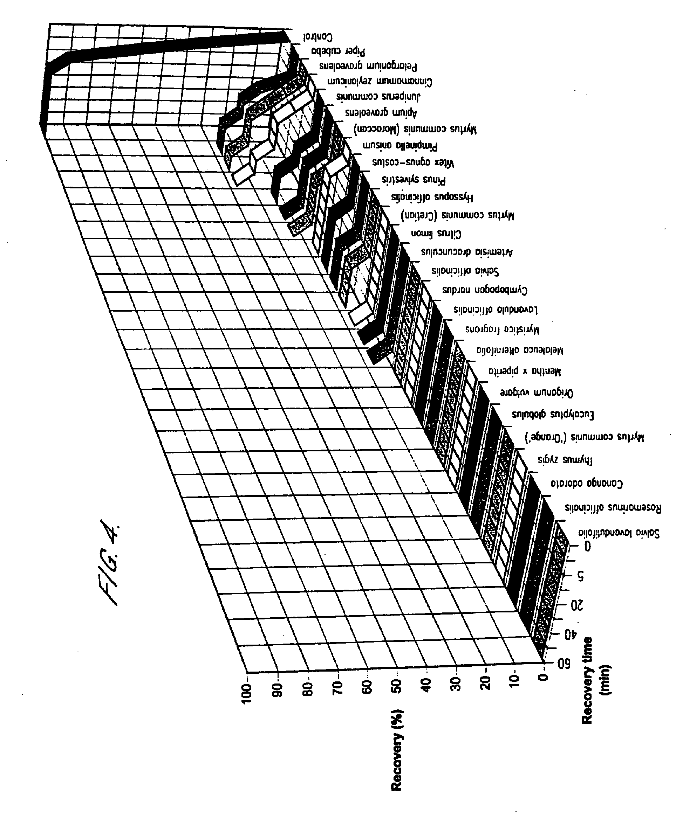

At 40 mgml⁻¹ (4 % w/v), lice showed no recovery when exposed to sage oil, or a number of other oils (FIG. 4), but, due to the greater activity at 2% (w/v), sage oil was clearly the most active oil tested.

### Example 2

Efficacy of a formulated product against parasites *in vivo.*

A formulated product containing 4% (w/v) Spanish sage essential oil and 0.5% *Aloe vera* gel (10x concentrated) in Lubrajel TW (1 part)-water (2 parts) was applied to the hair of a volunteer infested with head lice. After initial assessment of the infestation, 60 ml of the treatment was applied to the scalp, and covered with a shower cap for a period of 20 minutes. The cap was then removed, and the product removed by careful washing. The volunteer's hair was combed with a fine-toothed comb to remove dead lice. The procedure was then repeated after 7 days, to allow any eggs not killed by the initial treatment to be hatch. Following the second application, and after a period of 7 further days, there was no sign of lice infestation in the volunteer.

## Claims

1. A pharmaceutical composition comprising an essential oil in a gel carrier, said essential oil being obtained from a plant selected from the genera *Salvia, Artemisia, Citrus, Laurus, Myristica, Origanum, Piper* or *Aloysia,* said composition being for use in the treatment of a human or animal having a parasitic insect infestation.

2. A composition as claimed in claim 1 wherein the concentration of essential oil is from about 0.1 to about 50% w/v of the composition.

3. A composition as claimed in claim 2 wherein the concentration of essential oil is from about 0.1 to about 8% w/v of the composition.

4. A composition as claimed in claim 3 wherein the concentration of essential oil is about 4% w/v of the composition.

5. A composition as claimed in any preceding claim wherein the gel is based on agar, agarose, gelatin or a synthetic gelling agent.

6. A composition as claimed in claim 5 wherein the synthetic gelling agent is a gel based on glyceryl polymethacrylate and propylene glycol.

7. A composition as claimed in any one of claims 5 to 6 wherein the gelling agent contains about 0.1 to about 95%, preferably about 0.1 to about 66% water v/v and/or alcohol, preferably isopropyl alcohol (IPA) at about 0.1% to about 20% v/v.

8. A composition as claimed in any preceding claim wherein said essential oil is obtained from *Salvia lavandulifolia* or *Salvia officinalis.*

9. A composition as claimed in any one of claims 1 to 8 wherein said essential oil is obtained from a plant of the genera *Citrus*.

10. A composition as claimed in any one of claims 1 to 8 which includes essential oil obtained from the plant genera *Salvia* and *Citrus.*

11. A composition as claimed in claim 8 which comprises the essential oil of *Salvia lavandulifolia* at about 4% w/v dispersed in a gel diluted about 1:2 v/v with water.

12. A composition as claimed in any preceding claim comprising an anti-pruritic agent.

13. A composition as claimed in claim 12 wherein said anti-pruritic agent is *Aloe vera.*

14. A composition as claimed in claim 13 wherein said composition includes *Aloe vera* gel.

15. A composition as claimed in claim 14 which comprises about 0.1 to about 5.0% w/v *Aloe vera* gel.

16. A composition as claimed in any one of claims 11 to 15 which comprises about 0.5% w/v *Aloe vera* gel.

17. Use of a gel and essential oil selected from the genera *Salvia, Artemisia, Citrus*, *Juniperus, Laurus, Myristica, Origanum, Piper* or *Aloysia* in the manufacture of a pharmaceutical gel composition for the treatment of a human or animal having a parasitic insect infestation.

18. A composition as claimed in any one of claims 1 to 16 for use in the treatment of a human or animal having a parasitic insect infestation wherein said parasitic insects are selected from lice, lice eggs, mites, fleas or parasites associated with blowfly strike.

19. A composition as claimed in claim 18 for the use in the treatment of a human or animal having a parasitic infestation wherein said parasitic insects are selected from head lice (*Pediculus humanus capitis, syn. P. capitis*), clothing lice (*Pediculus humanus humanus syn. P. corporis),* pubic lice (*Pthirius pubis*), biting lice (*Bovicula* ovis), scab mite *(Psoroptes* ovis), ear mite *(Psoroptes cuniculi),* dust mites (primarily of the genus *Dermatophagoides,* pig mites, cat fleas *(Ctenocephalalides felis),* dog fleas (C. *canis*), horse fleas and *Lucilia* or *Chrysomya* species.

## Patentansprüche

1. Pharmazeutische Zusammensetzung, umfassend ein ätherisches Öl in einem Gelträger, wobei das ätherische Öl erhalten wird aus einer Pflanze, ausgewählt aus der Gattung *Salvia, Artemisia, Citrus, Laurus, Myristica, Origanum, Piper* oder *Aloysia,* wobei die Zusammensetzung zur Verwendung bei der Behandlung eines Menschen oder Tieres mit einem Befall durch parasitäre Insekten ist.

2. Zusammensetzung nach Anspruch 1, worin die Konzentration des ätherischen Öls von etwa 0,1 bis etwa 50 % Gew./V der Zusammensetzung ist.

3. Zusammensetzung nach Anspruch 2, worin die Konzentration des ätherischen Öls von etwa 0,1 bis etwa 8 % Gew./V der Zusammensetzung ist.

4. Zusammensetzung nach Anspruch 3, worin die Konzentration des ätherischen Öls etwa 4 % Gew./V der Zusammensetzung ist.

5. Zusammensetzung nach einem der vorhergehenden Ansprüche, worin das Gel auf Agar, Agarose, Gelatine oder einem synthetischen gelierenden Mittel basiert.

6. Zusammensetzung nach Anspruch 5, worin das synthetische gelierende Mittel ein Gel ist, das auf Glycerylpolymethacrylat und Propylenglykol basiert.

7. Zusammensetzung nach einem der Ansprüche 5 bis 6, worin das gelierende Mittel etwa 0,1 bis etwa 95 %, vorzugsweise etwa 0,1 bis etwa 66 % Wasser VN und/oder Alkohol, vorzugsweise Isopropylalkohol (IPA), von etwa 0,1 % bis etwa 20 % V/V enthält.

8. Zusammensetzung nach einem der vorhergehenden Ansprüche, worin das ätherische Öl erhalten wird aus *Salvia lavandulifolia* oder *Salvia officinalis.*

9. Zusammensetzung nach einem der Ansprüche 1 bis 8, worin das ätherische Öl erhalten wird aus einer Pflanze der Gattung *Citrus.*

10. Zusammensetzung nach einem der Ansprüche 1 bis 8, umfassend ätherisches Öl, erhalten aus der Pflanzengattung *Salvia* und *Citrus.*

11. Zusammensetzung nach Anspruch 8, umfassend das ätherische Öl von *Salvia lavandulifolia* in etwa 4 % Gew.N, dispergiert in einem Gel, etwa 1:2 V/V verdünnt mit Wasser.

12. Zusammensetzung nach einem der vorhergehenden Ansprüche, umfassend ein antipruritisches Mittel.

13. Zusammensetzung nach Anspruch 12, worin das antipruritische Mittel *Aloe vera* ist.

14. Zusammensetzung nach Anspruch 13, worin die Zusammensetzung *Aloe vera-*Gel enthält.

15. Zusammensetzung nach Anspruch 14, umfassend etwa 0,1 bis etwa 5,0 % Gew.N *Aloe vera-*Gel.

16. Zusammensetzung nach einem der Ansprüche 11 bis 15, umfassend etwa 0,5 % Gew.N *Aloe vera*-Gel.

17. Verwendung eines Gels und eines ätherischen Öls, ausgewählt aus der Gattung *Salvia, Artemisia, Citrus, Juniperus, Laurus, Myristica, Origanum, Piper* oder *Aloysia* bei der Herstellung einer pharmazeutischen Gelzusammensetzung zur Behandlung eines Menschen oder Tieres mit einem Befall durch parasitäre Insekten.

18. Zusammensetzung nach einem der Ansprüche 1 bis 16 zur Verwendung bei der Behandlung eines Menschen oder Tieres mit einem Befall durch parasitäre Insekten, worin die parasitären Insekten ausgewählt sind aus Läusen, Läuseeiern, Milben, Flöhen oder Parasiten, die assoziiert sind mit Schmeißfliegenbefall.

19. Zusammensetzung nach Anspruch 18 zur Verwendung bei der Behandlung eines Menschen oder Tieres mit einem parasitären Befall, worin die parasitären Insekten ausgewählt sind aus Kopfläusen (*Pediculus humanus capitis, syn. P. capitis*), Kleiderläusen (*Pediculus humanus humanus syn. P. corporis),* Filzläusen (*Pthirius pubis),* Kieferläusen (*Bovicula ovis),* Schorfmilben *(Psoroptes ovis),* Ohrmilben *(Psoroptes cuniculi*), Staubmilben (primär von der Gattung *Dermatophagoides),* Schweinemilben, Katzenflöhen *(Ctenocephalalides felis*), Hundeflöhen *(C. canis*), Pferdeflöhen und *Lucilia-* oder *Crysomya*-Spezies.

## Revendications

1. Composition pharmaceutique comprenant une huile essentielle dans un gel de support, ladite huile essentielle étant obtenue à partir d'une plante choisie parmi les genres *Salvia, Artemisia, Citrus, Laurus, Myristica, Origanum, Piper* ou *Aloysia*, ladite composition étant destinée à être utilisée dans le traitement d'un être humain ou d'un animal atteint d'une infestation par des insectes parasites.

2. Composition selon la revendication 1, dans laquelle la concentration d'huile essentielle est d'environ 0,1 à environ 50 % en poids/volume de la composition.

3. Composition selon la revendication 2, dans laquelle la concentration d'huile essentielle est d'environ 0,1 à environ 8 % en poids/volume de la composition.

4. Composition selon la revendication 3, dans laquelle la concentration d'huile essentielle est d'environ 4 % en poids/volume de la composition.

5. Composition selon l'une quelconque des revendications précédentes, dans laquelle le gel est à base de gélose, d'agarose, de gélatine ou d'un agent gélifiant synthétique.

6. Composition selon la revendication 5, dans laquelle l'agent gélifiant synthétique est un gel à base de polyméthacrylate de glycéryle et de propylèneglycol.

7. Composition selon l'une quelconque des revendications 5 à 6, dans laquelle l'agent gélifiant contient environ 0,1 à environ 95 %, de préférence environ 0,1 à environ 66 %, en volume d'eau et/ou un alcool, de préférence l'alcool isopropylique (IPA), à environ 0,1 % à environ 20 % en volume.

8. Composition selon l'une quelconque des revendications précédentes, dans laquelle ladite huile essentielle est obtenue à partir de *Salvia lavandulifolia* ou *Salvia officinalis.*

9. Composition selon l'une quelconque des revendications 1 à 8, dans laquelle ladite huile essentielle est obtenue à partir d'une plante du genre *Citrus*.

10. Composition selon l'une quelconque des revendications 1 à 8, qui comprend une huile essentielle obtenue à partir des genres végétaux *Salvia* et *Citrus.*

11. Composition selon la revendication 8, qui comprend l'huile essentielle de *Salvia lavandulifolia* à environ 4 % en poids/volume dispersée dans un gel dilué à environ 1:2 en volume avec de l'eau.

12. Composition selon l'une quelconque des revendications précédentes, comprenant un agent antiprurigineux.

13. Composition selon la revendication 12, dans laquelle ledit agent antiprurigineux est *Aloe vera.*

14. Composition selon la revendication 13, dans laquelle ladite composition comprend un gel de *Aloe vera.*

15. Composition selon la revendication 14, qui comprend environ 0,1 à environ 5,0 % en poids/volume de gel de *Aloe vera.*

16. Composition selon l'une quelconque des revendications 11 à 15, qui comprend environ 0,5 % en poids/volume de gel de *Aloe vera.*

17. Utilisation d'un gel et d'une huile essentielle choisie parmi les genres *Salvia, Artemisia, Citrus, Juniperus, Laurus, Myristica, Origanum, Piper* ou *Aloysia* dans la fabrication d'une composition de gel pharmaceutique destinée au traitement d'un être humain ou d'un animal atteint d'une infestation par des insectes parasites.

18. Composition selon l'une quelconque des revendications 1 à 16, à utiliser dans le traitement d'un être humain ou d'un animal atteint d'une infestation par des insectes parasites, lesdits insectes parasites étant choisis parmi les poux, les lentes, les acariens, les puces ou les parasites associés à une atteinte par les mouches à viande.

19. Composition selon la revendication 18, à utiliser dans le traitement d'un être humain ou d'un animal atteint d'une infestation parasitaire, dans laquelle lesdits insectes parasites sont choisis parmi les poux de la tête *(Pediculus humanus capitis, syn. P. Capitis),* les poux du corps (*Pediculus humanus humanus syn. P. Corporis),* les morpions *(Pthirius pubis*), les mallophages du mouton *(Bovicula ovis),* le psoropte du mouton *(Psoroptes ovis),* la mite d'oreilles *(Psoroptes cuniculi*), les acariens de la poussière (principalement du genre *Dermatophagoides*), les acariens du porc, les puces du chat *(Ctenocephalides felis),* les puces du chien *(C. canis),* les puces du cheval et les espèces de *Lucilia* ou *Chrysomya.*
